# EUROPEAN PATENT APPLICATION

(11) **EP 2 979 762 A1**
(43) Date of publication of application: **03.02.2016**
(21) Application number: 14775187.9
(22) Date of filing: 25.03.2014
(51) Int. Cl.: B01L 1/00, B25J 21/02, C12M 1/00

(54) **ISOLATOR SYSTEM**

(30) Priority: 29.03.2013 JP 2013071696
(71) Applicant: Panasonic Healthcare Holdings Co., Ltd., Tokyo 105-8433 (JP)
(72) Inventor: SEKINE, Hironobu, Ehime 791-0395 (JP); KOBAYASHI, Koichi, Ehime 791-0395 (JP); YOKOI, Yasuhiko, Ehime 791-0395 (JP)
(74) Representative: Grünecker Patent- und Rechtsanwälte PartG mbB
(86) International application number: PCT/JP2014/001725
(87) International publication number: WO 2014/156137

(57) **Abstract**

An isolator attached with gloves, includes: a main body case including a work space isolated from an exterior thereof; a work plate provided within the main body case, the work plate having formed therein an opening portion connecting a device provided below the main body case and the work space; a first cover member mounted to the work plate in a manner openable and closable with respect to the opening portion, the first cover member having formed therein a hole portion into which a worker' s finger is to be inserted; and a second cover member mounted to the first cover member in a manner openable and closable with respect to the hole portion.

## Description

### [Technical Field]

The present disclosure relates to an isolator used in laboratory environment equipment related to regenerative medicine and pharmaceutical production.

### [Background]

Patent Literature 1 discloses a centrifuge provided below a work plate. This centrifuge is covered with an openable and closable cover portion. The cover portion is a part of a work plate and is mounted flash with the work plate.

### [Citation List]

### [Patent Literature]

Japanese Patent Application Laid-Open Publication No. 2011-177091

### [Summary]

### [Technical Problem]

For example, an operation of opening/closing a cover portion in Patent Literature 1 is conducted by a worker's hands using gloves. However, since the cover portion is mounted flash with a work plate, the operation of opening/closing the cover portion may become difficult. Then, the present disclosure is to provide an isolator whose operability is improved.

### [Solution to Problem]

An isolator attached with gloves in the present disclosure includes:
a main body case including a work space isolated from an exterior thereof;
a work plate provided within the main body case, the work plate having formed therein an opening portion connecting the work space and a device provided below the main body case;
a first cover member mounted to the work plate in a manner openable and closable with respect to the opening portion, the first cover member having formed therein a hole portion into which a worker's finger is to be inserted; and
a second cover member mounted to the first cover member in a manner openable and closable with respect to the hole portion.

### [Advantageous Effects]

The isolator in the present disclosure is effective in improving its operability.

### [Brief Description of the Drawings]

Fig. 1 is a front view illustrating an isolator system according to a first embodiment.
Fig. 2 is a perspective view illustrating an isolator system according to a first embodiment.
Fig. 3 is a perspective view illustrating an isolator system when an incubator is mounted according to a first embodiment.
Fig. 4 is a schematic view illustrating a configuration of a work platform according to a first embodiment.
Fig. 5 is a schematic view illustrating configurations of cover members and a hinge according to a first embodiment.
Fig. 6 is a schematic view illustrating configurations of cover members and a hinge according to a first embodiment.
Fig. 7 is a schematic view illustrating operations of cover members and a hinge according to a first embodiment.
Fig. 8 is a schematic view illustrating operations of cover members and a hinge according to a first embodiment.
Fig. 9 is a schematic view illustrating operations of cover members and a hinge according to a first embodiment.
Fig.10 is a schematic view illustrating operations of cover members and a hinge according to a first embodiment.
Fig.11 is a diagram illustrating a glove according to a first embodiment.
Fig.12 is a schematic view illustrating configurations of cover members and a hole portion according to another embodiment.
Fig. 13 is a schematic view illustrating configurations of cover members and a hinge according to another embodiment.
Fig. 14 is a schematic view illustrating configurations of cover members and a hinge according to another embodiment.
Fig. 15 is a schematic view illustrating configurations of cover members and a hinge according to another embodiment.
Fig. 16 is a schematic view illustrating configurations of cover members and a hinge according to another embodiment.

### [Mode for Carrying Out the Disclosure]

Hereinafter, embodiments will be described in detail with reference to drawings as necessary. However, more detailed description than necessity may be omitted. For example, the detailed descriptions of matters which have already well known and the repeated descriptions of substantially the same configurations may be omitted. This is because the following description is avoided from being redundant more than necessary, and facilitates the understanding by a person skilled in the art.

Note that the invertors provide the accompanying drawings and the following description to help a person skilled in the art fully understand the present disclosure, and are not intended to limit the subject matters described in the scope of claims thereby.

### (First Embodiment)

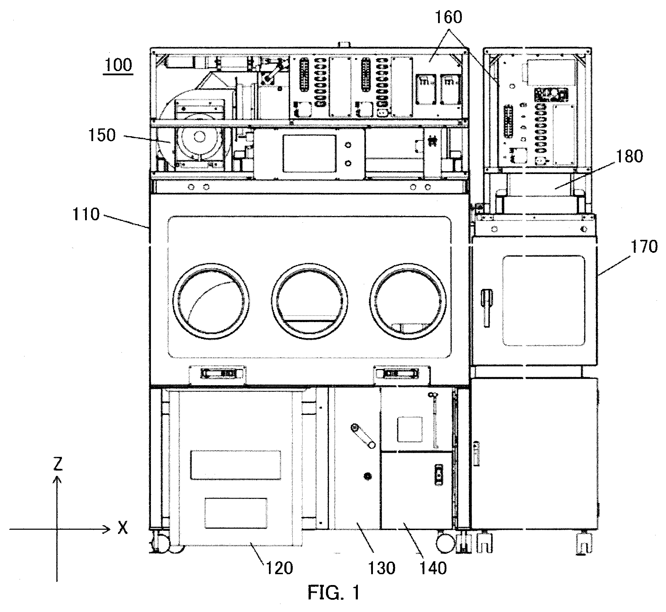

An isolator system 100 will be described hereinafter as an example of an isolator system in a first embodiment with reference to Figs. 1 to 10.

The isolator system 100 in the first embodiment is a device configured to perform, for example, work for cell culture, manipulation, observation, etc., in a sterilized environment. Sterilizing refers to an act of killing microorganisms, cells, etc., to bring a state closer to a sterile environment.

Note that, in the present embodiment, the Z-axis is an axis along a vertical direction in which the isolator system 100 is provided to stand, and it is assumed that a direction toward the upper side is +Z-direction and a direction toward the lower side (downward) is -Z-direction. The Y-axis is an axis along a direction perpendicular to the front surface and the back surface of the isolator system 100, and it is assumed that a direction extending from the front surface, where opening portions for conducting work within a work space is provided, to the back surface opposite to the front surface is -Y-direction, and a direction extending from the back surface to the front surface is +Y-direction. The X-axis is an axis along a direction perpendicular to the side surfaces on the left and right sides when seen from the front, and it is assumed that a direction extending from the left side surface to the right side surface when seen from the front is +X-direction, and a direction extending from the right side surface to the left side surface is -X-direction.

### [1. Configuration]

### [1-1. Isolator System]

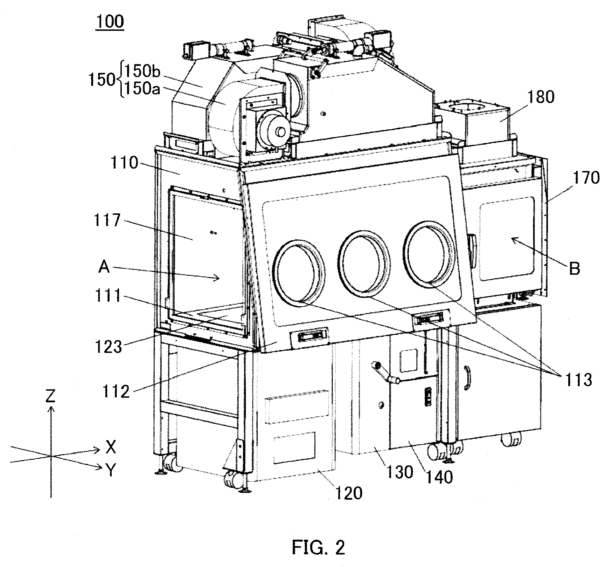

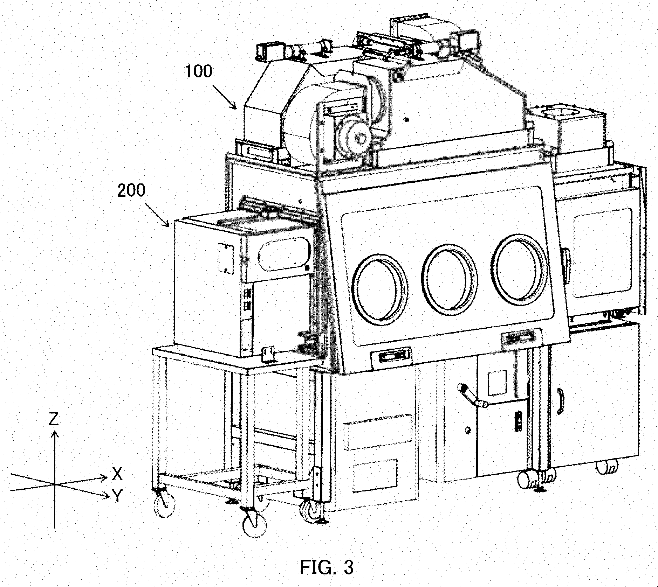

The overall configuration of the isolator system 100 will be described with reference to Figs. 1 to 4. Figs. 1 and 2 are a front view and a perspective view of the isolator system 100 according to the first embodiment, respectively, and Fig. 3 is a perspective view of the isolator system 100 when an incubator 200 is mounted according to the first embodiment.

As illustrated in Fig. 1, the isolator system 100 according to the first embodiment includes: an isolator 110; a centrifuge unit 120; an observation unit 130; a sterilizing unit 140; an air conditioning unit 150; a control unit 160; a pass box 170; and an air conditioning unit 180.

As illustrated in Fig. 2, the isolator 110 includes a substantially box-shaped work space A that is isolated from the surroundings. The detailed configuration thereof will be described later. The centrifuge unit 120 is provided below the isolator 110, and can be connected from the work space A. The centrifuge unit 120 includes, in the interior thereof, a centrifuge configured to centrifuge a sample to be worked within the work space A. The observation unit 130 is provided below the isolator 110, and can be connected from the work space A. The observation unit 130 includes, in the interior thereof, an observation device configured to observe a sample to be worked on within the work space A. Further, the observation unit 130 includes : an elevating mechanism capable of raising and lowering the observation device provided therewithin; and a handle provided outside with which the elevating mechanism is operated. A worker operates the elevating mechanism with the handle, thereby being able to lift the observation device into the work space A when the observation device is used, and accommodate the observation device in the observation unit 130 when the observation device is not used. The sterilizing unit 140 is provided below the isolator 110 in order to sterilize the interior of the isolator 110. The sterilizing unit 140 is configured to spray a sterilizing mist, obtained by converting a sterilizing liquid into mist, through a nozzle 143 provided within the isolator 110, and sterilize the interior thereof.

The air conditioning unit 150 is provided above the isolator 110 and is configured to control the air conditioning therewithin. The air conditioning unit 150 includes an intake unit 150a and a discharge unit 150a. The air conditioning unit 150 includes an inlet 151 and an outlet 155 provided on an upper surface plate in the isolator 110. The air is supplied into the isolator 110 from the inlet 151, and is discharged from the outlet 155. In the isolator 110, in order to secure a sterile environment in the interior thereof, a particulate trap filter of a HEPA filter 152 is provided to the inlet 151, and the air is supplied through the particulate trap filter into the isolator 110. A HEPA filter 156 is also provided to the outlet 155, and the gas within the isolator 110 is discharged from the interior of the isolator 110 through the HEPA filter 156. In the isolator 110, a sterilizing substance such as hydrogen peroxide is sprayed thereinto, and thereby sterilizing processing of sterilizing the interior of the isolator 110 is performed.

The control unit 160 is provided above the isolator 110 and the pass box 170, and is configured to control the operations of devices such as the sterilizing unit 140, the air conditioning unit 150, a plurality of diffusion fans 190, which will be described later, and the like.

The pass box 170 is provided on the side surface of the isolator 110 so that a worker puts a work item into the work space A from the outside. In the interior of the pass box 170, a transport space B for temporarily storing the work item is formed. The transport space B has hermeticity against the surrounding environment. Before the work item is put into the work space A from the outside, the work item is sterilized within the transport space B. In the side surface of the pass box 170, an opening for moving the work item is provided, and the isolator 110 and the pass box 170 are fixed so that the opening in the side surface of the pass box 170 is opposed to an opening provided in the side surface of the isolator 110. Thus, the work space A and the transport space B communicate with each other with hermeticity being maintained. A door that is openable and closable is mounted to the opening of the pass box 170. The door can separate the transport space B and the work space A with hermeticity being maintained. The air conditioning unit 180 is provided above the pass box 170 and is configured to control the air conditioning within the transport space B.

As illustrated in Fig. 2, in the isolator system 100, the incubator 200 is mounted on the side surface opposite to the side surface of the isolator 110 to which the pass box 170 is provided. The incubator 200 includes a storage chamber (not illustrated) in the interior thereof. The storage chamber is a chamber in which a culture is stored, and is partitioned as a space that restrains bacterial invasion from the outside by, for example, a rectangular parallelepiped-shaped box body. The storage chamber is partitioned with, for example, stainless steel plates. The incubator 200 is demountably configured with respect to the isolator system 100. As a result, culture can be controlled in each incubator 200. For example, a dedicated incubator 200 is used for each donor, and thus it is possible to restrain occurrence of failures such as mix-ups of cultures.

### [1-2. Isolator]

The configuration of the isolator 110 will be described with reference to Fig. 2.

As illustrated in Fig. 2, the isolator 110 is partitioned by a front surface plate 112 provided with a plurality of front surface opening portions 113, a bottom surface plate, an upper surface plate and side surface plates on the left and right sides, and includes, in the interior thereof, a work platform 111 used for conducting work; and a partition plate 117. The work space A in a box shape, which is a space for conducting work, is partitioned by the work platform 111, the partition plate 117, the upper surface plate, and the left and right side surface plates. The isolator 110 is partitioned to have hermeticity so that bacterial invasion from the outside is restrained. A duct for discharging the gas in the work space A is formed between the work platform 111 and the bottom surface plate and between the back surface plate and the partition plate 117. In the isolator 110 of the present embodiment, the work platform 111, the partition plate 117, the bottom surface plate, the back surface plate, the upper surface plate, and the left and right side surface plates are configured with stainless steel plates, which are easily cleaned and sterilized.

The front surface plate 112 is configured with a glass plate, and the front surface thereof is provided with a plurality of front surface opening portions 113 that are insertion portions for workers' hands. A glove 400 is mounted in each of the plurality of front surface opening portions 113. The front surface plate 112 is openable and closable with a hinge, provided in an upper end portion, as an axis. Thus, the front surface of the isolator 110 can be opened and closed. In the left and right side surface plates, openings for mounting the pass box 170 and the incubator 200 are provided. In the isolator 110, the pass box 170 is mounted to the opening of the right side surface plate, and a door for a delivery opening of the pass box 170 constitutes a part of the right side surface plate. Further, the incubator 200 is mounted to the left opening of the left side surface plate, and a door of the incubator 200 constitutes a part of the left side surface plate. In working, the worker conducts work in the work space A through the gloves. A Hanging bar, on which items used for work are hung, is provided on the upper surface plate.

### [1-3. Work Platform]

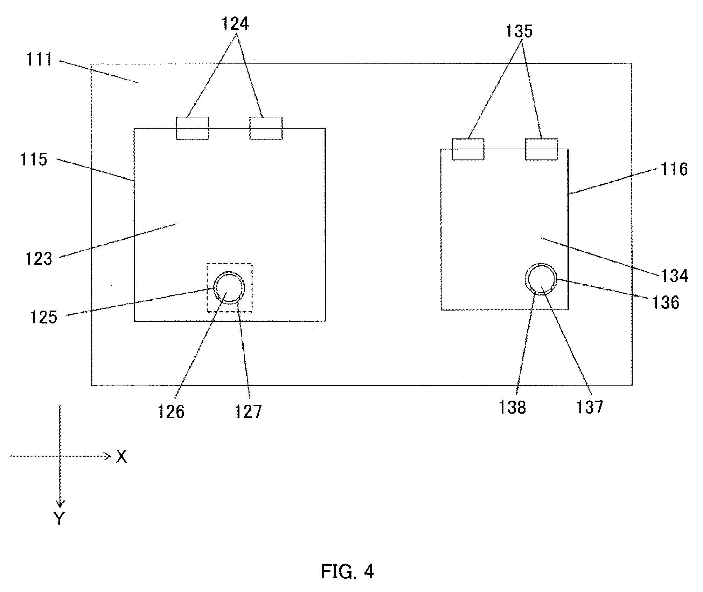

The configuration of the work platform 111 will be described with reference to Figs. 4 to 6. Fig. 4 is a schematic view illustrating a configuration of the work platform 111 according to a first embodiment.

In the work platform 111, a connection opening portion 115 for connecting to a centrifuge unit 120 provided below the work platform 111 and a connection opening portion 116 for connecting to the observation unit 130 provided below the work platform 111 are formed. The work platform 111 is mounted, via a hinge 124, with a cover 123 configured to be openable and closable with respect to the connection opening portion 115 and constitute a part of the work platform 111. The cover 123 is configured to be opened upward and closed through the hinge 124 with the hinge 124 used as an axis. In the cover 123, a hole portion 125 is formed at which a worker hooks his/her finger when opening the cover 123 by pulling it upward. Further, the cover 123 is provided, through a hinge 127, with a cover 126 (also referred to as a "cover member 126") configured to constitute a part of the work platform 111 and be openable and closable with respect to the hole portion 125. Note that the cover 123 and the cover 126 are provided on substantially the same plane as the work platform 111.

Similarly, the work platform 111 is mounted, via a hinge 135, with a cover 134 configured to constitute a part of the work platform 111 and be openable and closable with respect to the connection opening portion 116. The cover 134 can be opened upward and closed through the hinge 135 with the hinge 135 used as an axis. In the cover 134, a hole portion 136 is formed at which a worker hooks his/her finger when opening the cover 134 by pulling it upward. Further, the cover 134 is provided, via a hinge 138, with a cover 137 (also referred to as a "cover member 137") configured to constitute a part of the work platform 111 and be openable and closable with respect to the hole portion 136. Note that the cover 134 and the cover 137 are provided on substantially the same plane as the work platform 11.

With this configuration, when using the centrifuge unit 120, a worker hooks his/her finger at the hole portion 125 from the work space A, and opens the cover 123 upward to connect to the centrifuge unit 120. Similarly, when using the observation unit 130, a worker hooks his/her finger at the hole portion 136 from the work space A, opens the cover 134 upward to connect to the observation unit 130, and elevates/lowers the observation device within the observation unit 130 by the elevating means.

### [1-4. Cover Member]

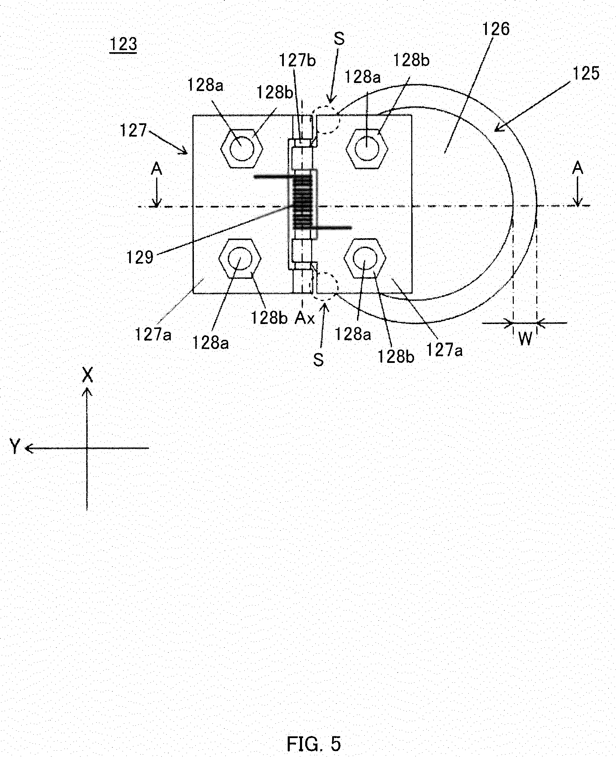

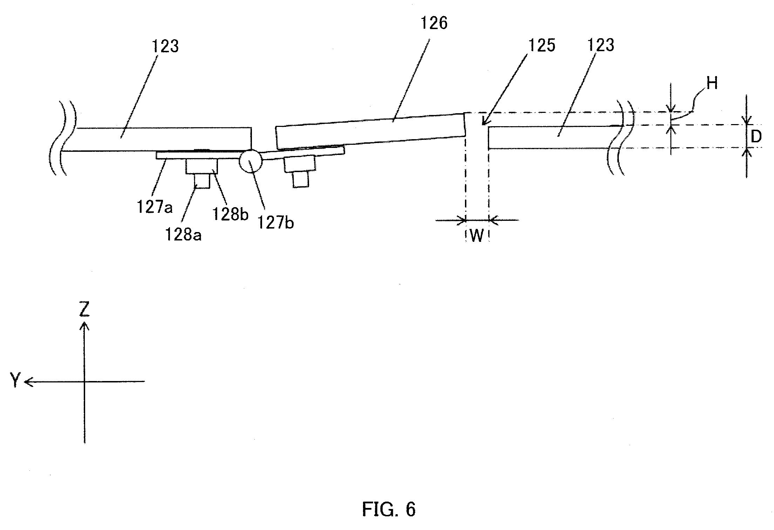

The detailed configurations of the cover members 126 and 137 will be described with reference to Figs. 5, 6 and 11. Figs. 5 and 6 are schematic views illustrating the cover member 126 and the hinge 127 according to the first embodiment. Note that since the configurations of the cover 137 and the hinge 138 are similar to those of the cover 126 and the hinge 127, the descriptions thereof are omitted herein. Note that, in Fig. 5, for example, one mounted to the cover 123 out of two mounting portions 127a corresponds to a first portion, while the other one mounted to the cover 126 corresponds to a second portion. Further, the cover 126 may be formed using, for example, a laser beam machine.

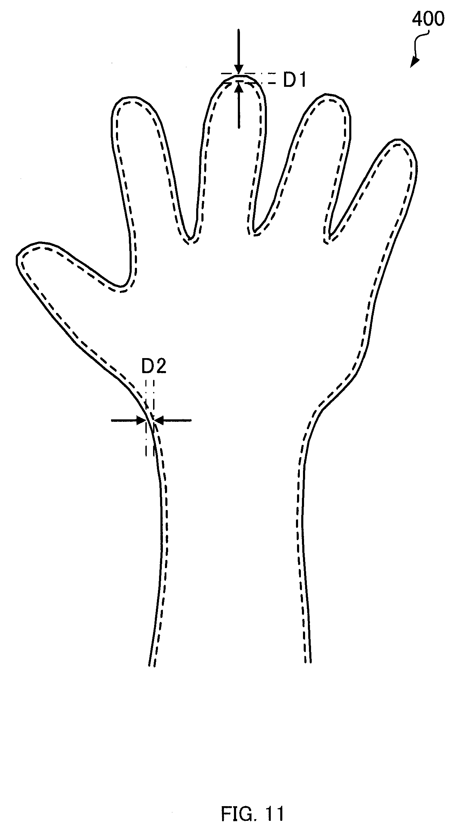

As illustrated in Fig. 5, two bolts 128a are welded to the back surfaces of the cover 123 and the cover 126, respectively. The bolts 128a fit into the hinge 127, and the hinge 127 is fixed to the back surfaces (-Z) of the cover 123 and the cover 126 using nuts 128b. The hinge 127 includes, for example, the two mounting portions 127a fixed to the back surfaces of the cover 123 and the cover 126, respectively; an axis 127b configured to couple the mounting portions 127a in a rotatable manner; and a spring 129 mounted to the axis 127b and configured to push the mounting portions 127a. Thus, the mounting portion 127a fixed to the cover 126 can pivot about the axis 127b as a pivot axis, with respect to the mounting portions 127a fixed to the cover 123. Further, the spring 129 is configured to push the mounting portions 127a toward the back surfaces of the cover 123 and the cover 126 with the axis 127b as an axis. That is, the cover 126 is pushed upward through the hinge 127 with respect to the cover 123, that is, to the surface side of the cover 123. Further, in the mounting portion 127a fixed to the cover 126, a restricting portion S, which is a part adjacent to the axis 127b, abuts against the cover 123 without abutting against the cover 126. Thus, the rotational movement of the mounting portion 127a fixed to the cover 126 is restricted, and the steady-state position of the cover 126 is determined. Here, the cover 126 is arranged, in a steady-state position, at least in such a position that an opening width W between an end portion thereof opposite to the pivot axis Ax and an edge portion of the hole portion 125 is greater than three times the thickness D1 (Fig. 11) of the glove 400 which is to be mounted to each of the front surface opening portions 113. In the present embodiment, the thickness D1 in fingertips of the glove 400 is 0.8 mm, and the opening width W is 4.0 mm. Thus, the opening width W is greater than three times the thickness D1 of the glove 400.

Here, Fig. 11 is a diagram illustrating a glove according to the first embodiment. Note that, in Fig. 11, an external form of the glove 400 is indicated by solid line, while an internal form of the glove 400 is indicated by dashed line, for the purpose of illustration. The glove 400 is formed such that the thickness D1 (i.e., 0.8 mm) in fingertips becomes greater than the thickness D2 (i.e., 0.4 mm) in, for example, the palm and wrist, etc. , other than the fingertips. Note that the thicknesses D1, D2 represent the thicknesses between the internal side and the external side in the glove 400.

Further, as illustrated in Fig. 6, the cover 126 is mounted to the cover 123 such that at least the upper surface end portion opposite to the pivot axis Ax of the cover 126 is positioned above the upper surface of the cover 123. In the present embodiment, the cover 126 is mounted to the cover 123 such that the upper surface end portion opposite to the pivot axis Ax of the cover 126 is positioned above the upper surface of the cover 123 by a height H. Further, the cover 126 is mounted to the cover 123 such that the lower surface end portion opposite to the pivot axis Ax of the cover 126 is positioned below the upper surface of the cover 123. That is, the cover 126 is mounted to the cover 123 such that the height H, at which the upper surface end portion opposite to the pivot axis Ax of the cover 126 is positioned above, is not greater than the thickness of the cover 126. The height H can be adjusted by changing the position and the space of the restricting portion S since backlash exists between the mounting portions 127a and the axis 127b. In the present embodiment, the cover 123 and the cover 126 are constituted by the same member, and the thickness D of the cover 123 and the thickness of the cover 126 are the same, and thus the height H is made smaller than the thickness D. Specifically, the thickness D is about 1.5 mm, whereas the height H is 0.5 mm.

### [2. Operation]

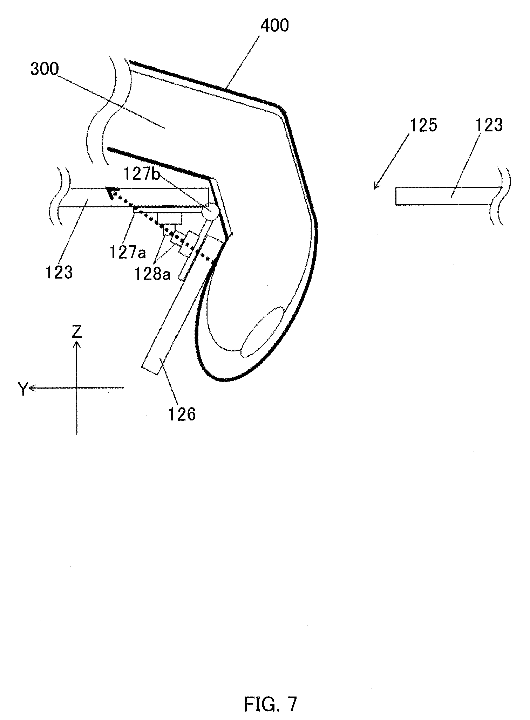

An operation of connecting from the work space A to the centrifuge unit 120 by a worker will be described with reference to Figs. 7 to 10. Figs. 7 to 10 are schematic diagrams illustrating operations of the cover members and the hinge according to the present embodiment. Here, an operation of opening the cover 123 will be described, however, since an operation of closing the cover 123 is basically the same, description thereof is omitted. Further, connecting to the observation unit 130 is similar thereto, description is omitted.

First, in an operation of connecting from the work space A to the centrifuge unit 120, a worker is required to pull the cover 123 upward and open it. Thus, as illustrated in Fig. 7, the worker pushes the upper surface of the cover 126 downward from a steady state (Fig. 6) with a finger 300, and inserts the finger 300 into the hole portion 125. Then, the worker hooks the finger 300 at the edge portion on the near side of the hole portion 125. Then, the worker exerts a force on the upper surface of the cover 126 pushed with the finger, and pulls the cover 123 upward. At this time, the cover 126 pushed downward via the hinge 127 is stopped with the bolts 128a provided to the lower surfaces abut against each other. Specifically, the bolts 128a provided on the lower surface of the cover 123 and the bolts 128a provided on the lower surface of the cover 126 abutting against each other. Thus, the cover 126 is restrained at the predetermined angle with respect to the cover 123. Therefore, the worker can securely apply a force onto the upper surface of the cover 126 with a fingertip. In the present embodiment, the cover 126 is restrained at a position forming an angle of about 60 degrees with respect to the cover 123.

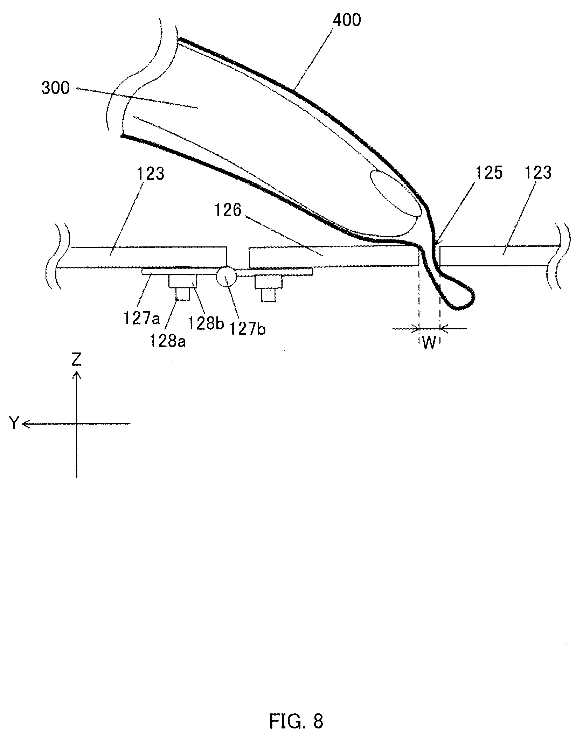

Thereafter, the worker is required to pull out the finger 300 from the hole portion 125. As illustrated in Fig. 8, in a situation where the worker pulls out the finger 300, when the finger 300 is released from the cover 126, the cover 126 is returned, by the hinge 127, to the position at which the cover 126 constitutes a part of the work platform 111. At this time, the glove 400 and fingers are not closely contact with each other, but there may be a space created between the glove 400 and fingers. Thus, even if the worker pulls out the finger 300 from the hole portion 125, the glove 400 may be caught between the cover 126 and the edge portion of the hole portion 125.

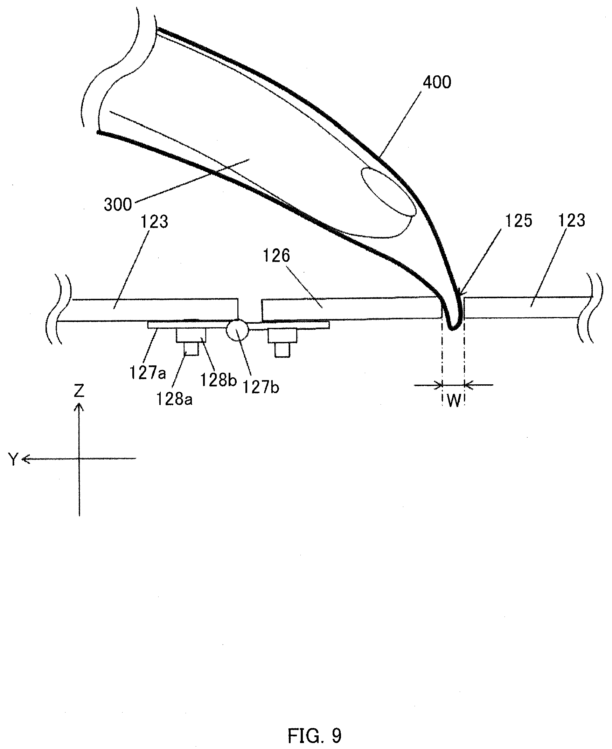

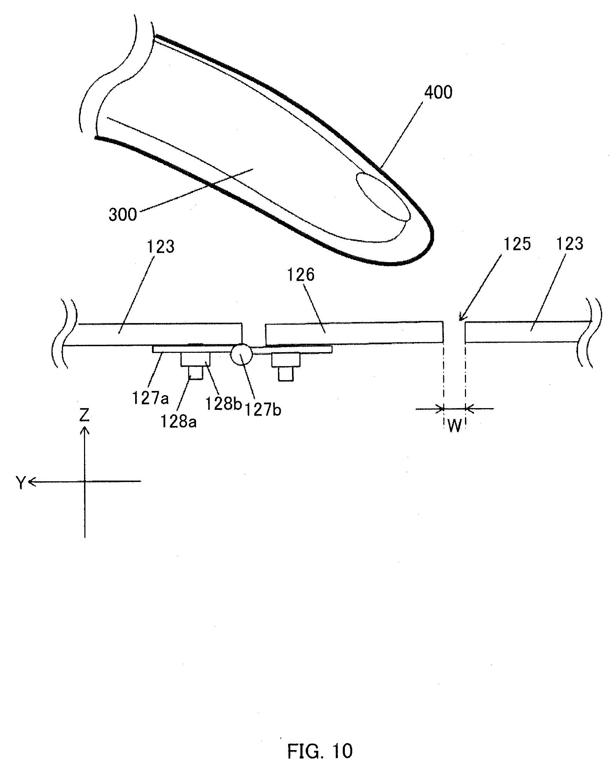

However, in the isolator 110, the cover 126 is arranged at such a position that the opening width W between the end portion opposite to the pivot axis Ax of the cover 126 and the edge portion of the hole portion 125 becomes greater than three times the thickness of the glove 400. Thus, as illustrated in Figs. 9 and 10, even if the glove 400 is caught between the cover 126 and the edge portion of the hole portion 125, the worker can pull out the glove 400 from between the cover 126 and the edge portion of the hole portion 125.

Here, an embodiment in which the opening width W is changed will be described with reference to Table 1. The glove 400 having a thickness of 0.8 mm is used in the embodiment. In Table 1, "operability" indicates the ease of pulling out the finger 300, "falling problem" indicates a problem of falling of an article used for work and the like, and "evaluation" indicates an evaluation as a product with the operability and the falling problem being taken into consideration.

As illustrated in Table 1, when the opening width W is 2.0 mm, the glove 400 can be pulled out although not smoothly. When the opening width W is equal to or greater than 3.0 mm, the glove 400 can be pulled out smoothly, and when the opening width W is equal to or greater than 4.0 mm, it can be pulled out without awkwardness. As such, the greater the opening width W becomes, the more the operability is improved.

Further, when the opening width W is equal to or smaller than 5.0 mm, problems of falling of an article, etc., hardly occur, but when the opening width W is equal to or greater than 6.0 mm, the possibility of falling of an article, etc., is increased. As such, the reduction in the opening width W reduces the occurrence of the falling problems, and thus is preferable.

According to the results described above, the inventors estimate that, when the glove has a thickness of 0.8 mm, it is preferable from the viewpoint of the operability that the opening width W is equal to or greater than three times the thickness of the glove 400, and it is preferable from the viewpoint of the falling problems that the opening width W is equal to or smaller than 5.0 mm.

**[Table 1]**

| Opening Width W (mm) | Operability | Falling Problem | Evaluation |
|---|---|---|---|
| 1.0 | × | ⊚ | × |
| 2.0 | Δ | ⊚ | Δ |
| 3.0 | ○ | ○ | ○ |
| 4.0 | ⊚ | ○ | ⊚ |
| 5.0 | ⊚ | ○ | ○ |
| 6.0 | ⊚ | Δ | Δ |
| 7.0 | ⊚ | × | × |
| 8.0 | ⊚ | × | × |

### [3. Effects, etc.]

As described above, in the present embodiment, an isolator system 100 (an example of the isolator) includes the work space A isolated from the exterior thereof, and includes a substantially box-shaped isolator 110 (an example of a main body case) having a plurality of front surface opening portions 113, to which the gloves 400 for inserting work hands are mounted, formed in the front face thereof. The isolator 110 includes the work platform 111 (an example of a work plate) for conducting work. In the work platform 111, formed is the connection opening portion 115(an example of the connection opening portion) for connecting from the work space A to, for example, the centrifuge unit 120 provided below the work platform 111. And the work platform 111 includes the cover 123 (an example of a first cover member) which is configured to constitute a part of the work platform 111 and with which the connection opening portion 115 can be opened and closed. In the cover 123, the hole portion 125 (an example of the hole portion) is formed, at which a worker hooks his/her finger to pull the cover 123 up and open it, and the cover 123 is provided, via the hinge 127 (an example of an elastic member), with the cover 126 (an example of a second cover member) which is configured to constitute a part of the work platform 111 and with which the hole portion 125 can be opened and closed. The cover 126 can be pushed downward by a worker with the finger 300, by way of the hinge 127, with the front side of the cover 126 (an example of one end side) as the pivot axis Ax, and when the worker's finger 300 is released, it returns to a position at which the cover constitutes a part of the work platform 111. That is, when the worker's finger 300 is released from the cover 126, the cover 126 returns to substantially the same plane as the cover 123. Moreover, the cover 126 is arranged at least such that the opening width W between the end portion (an example of an other side) on the back side of the 126 (an example of the other side) and the edge portion of the hole portion 125 becomes equal to or greater than three times the thickness of the glove 400 mounted to each of the front surface opening portions 113.

Thus, the worker can hook the finger 300 at the hole portion 125 only by pushing the cover 126 downward with the single finger 300, even if his/her hand is inserted into the glove 400. Therefore, the worker can easily open the cover 123 with only the single finger 300. Further, also when pulling out the finger 300 from the hole portion 125, the glove 400 can be restrained from being caught between the hole portion 125 and the cover 126 and not being able to be pulled out. Thus, the isolator system 100 can improve worker's operability.

Further, in the present embodiment, the cover 126 is mounted via the hinge 127 to the cover 123 at least such that the upper surface end portion on the back side of the cover 126 is positioned above the upper surface of the cover 123.

Thus, an article, etc., such as laboratory equipment, a sample, etc. , to be used for work can be restrained from rolling over and staying at the upper surface of the cover 126. Therefore, an article, etc., are retrained from falling into the hole portion 125 by mistake, when the worker pushes the cover 126 downward with the finger 300. Therefore, the isolator system 100 can improve worker's operability.

Further, in the present embodiment, the cover 126 is mounted via the hinge 127 to the cover 123 such that the lower surface end portion on the back side of the cover 126 is positioned below the upper surface of the cover 12.

As a result, the cover 126 constituting a part of the work platform 111 does not greatly protrude from the upper surface of the cover 123, and thus an article, etc. , such as laboratory equipment and a sample, etc., can be restrained form being caught by the cover 126 during the work on the work platform 111. Therefore, the isolator system 100 can improve worker's operability. Further, in the present embodiment, the lower surfaces of the cover 123 and the cover 126 are provided with the bolts 128a which are configured to restrain the rotational movement of the cover 126 with respect to the cover 123. Here, in the present embodiment, the bolts 128a are provided to each of the lower surfaces of the cover 123 and the cover 126, but a restraint portion configured to restrain the rotational movement of the cover 126 with respect to the cover 123 may be provided to at least one of the cover 123 and the cover 126.

Thus, the cover 126 pushed downward via the hinge 127 is stopped at the predetermined position by a restraint portion provided to the lower surface. In the present embodiment, the bolts 128a provided to the lower surface of the cover 123 and the bolts 128a provided to the lower surface of the cover 126 abut against each other, and the cover 126 is stopped at the predetermined angle with respect to the cover 123. Thus, a worker can securely apply a force to the upper surface of the cover 126 with a finger. In the present embodiment, the cover 126 is stopped at a position forming an acute angle of about 60 degrees with respect to the cover 123, but the acute angle with respect to the cover 123 is effective if it is smaller than about 90 degrees. The acute angle with respect to the cover 123 is more effective when it is equal to or greater than 45 degrees and equal to or smaller than 75 degrees.

Further, in the present embodiment, the bolts 128a also serve as the parts for fixing the hinge 127 to the lower surfaces of the cover 123 and the cover 126.

Thus, a restraint portion for restraining the rotational movement of the cover 126 with respect to the cover 123 is not separately provided, and thus design is facilitated and also there is an advantage in costs. It is effective if a restraint portion configured to restrain the rotational movement of the cover 126 with respect to the cover 123 and a part for fixing the hinge 127 to the lower surfaces of the cover 123 and the cover 126 are a bolt and a nut. This is because design is facilitated and also there is an advantage in costs.

### (Other Embodiment)

As described above, the first embodiment has been described as exemplification of technologies disclosed in the present application. However, technologies in the present disclosure are not limited thereto, and can be applied to embodiments to which modification, replacement, addition, omission and/or the like is made as necessary. Further, it is also possible to provide new embodiments by combining the constituent elements described in the above first embodiment.

Thus, another embodiment will be exemplified, hereinafter.

In the present embodiment, the hinge 127 and the hinge 138 are described as examples of an elastic member. However, technologies in the present disclosure are not limited thereto. The elastic member may only be such that the cover 126 can be pushed downward by a worker with the finger 300 with one end side of the cover 126 as the pivot axis Ax and that the cover 126 can return to a position at which it constitutes a part of the work platform 111 when being released from the worker's finger 300. Thus, a high elastic member such as rubber having a restoring force, synthetic resins, etc., may be used as an elastic member.

### <Center of Cover and Hole Portion>

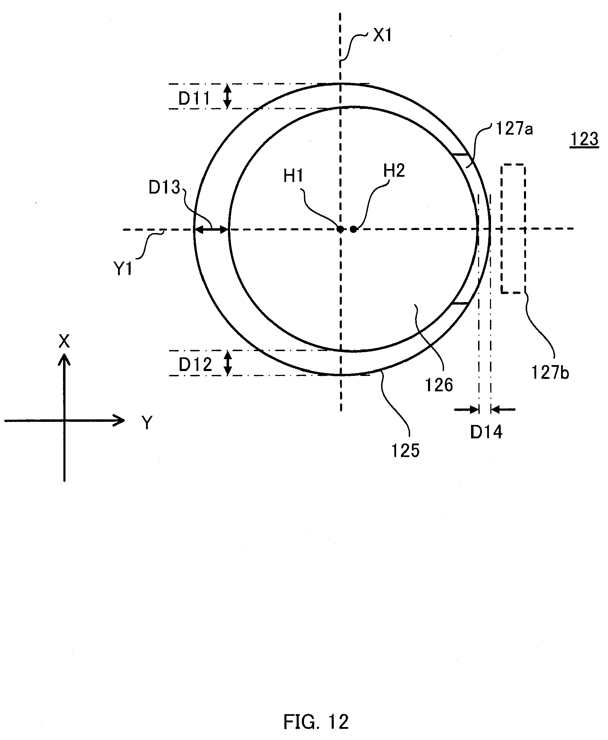

Further, the cover 126 and the hole portion 125 (first embodiment) are not limited to those formed substantially in a circular shape as illustrated in Fig. 5 and provided such that both the center thereof substantially coincide each other. For example, as illustrated in Fig. 12, the cover 126 may be mounted to the cover 123 such that the center H2 of the cover 126 and the center H1 of the hole portion 125 do not coincide with each other.

Fig. 12 is a schematic diagram illustrating configurations of covers and a hole portion. Fig. 12 illustrates the cover 123 and the cover 126 when seen from the upper side (+Z) to the lower side (-Z). Note that although the axis 127b cannot be seen, it is indicated by dashed line for the purpose of illustration. Further, a first line X1, which passes through the center H1 of the hole portion 125 and is parallel with the x-axis, and a second line Y2, which passes through the center H1 and is parallel with the Y-axis, are indicated by dashed line for the purpose of illustration.

The cover 126 in Fig. 12 is mounted to the cover 123 such that the center H2 of the cover 126 is provided on the axis 127b side (+Y) in the Y-axis direction with respect to the center H1 of the hole portion 125. Note that the cover 126 may be mounted such that gap widths D11, D12, D13 and D14 between the cover 126 and the cover 123 are 2.5 mm, 2.5 mm, 4 mm and 1 mm, respectively. With this configuration, it becomes possible to reliably prevent the glove 400 from being caught and to prevent an article used for work from falling down from the hole portion 125, when the cover 126 is opened/closed.

### <Shape of Cover>

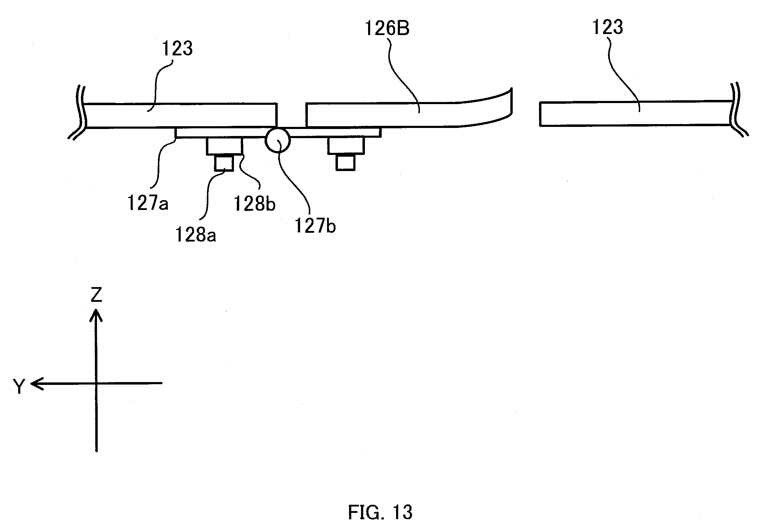

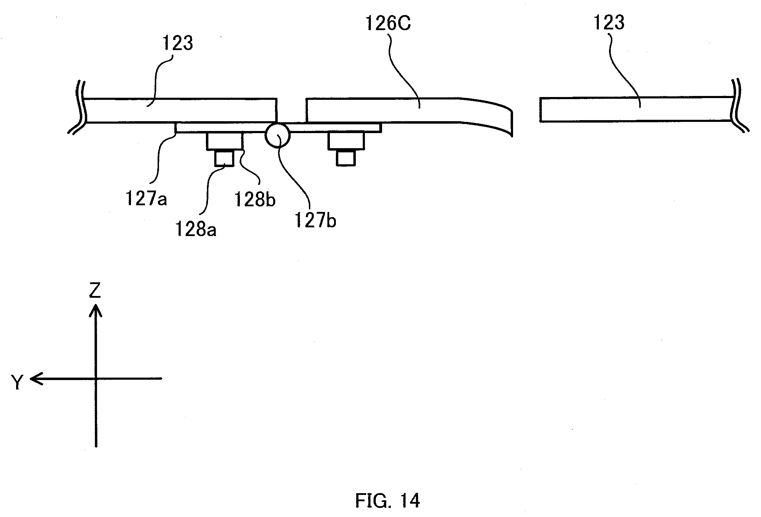

Further, the cover 126 (first embodiment) is not limited to such a cover as to be formed of a flat plate shape as illustrated in Fig. 6. For example, it may be formed of such a curved shape as a cover 126B (Fig. 13) and a cover 126C (Fig. 14) which are equivalent to the cover 126. Figs. 13 and 14 are schematic diagrams illustrating configurations of covers and a hinge according to other embodiments. Note that similar reference numerals are given to the constituents in Figs. 13 and 14 which are similar to the constituents illustrated in Fig. 6, and the descriptions thereof are omitted.

The cover 126B (Fig. 13) is formed of such a curved shape that the end portion on -Y side in the cover 126B is provided above (+Z) the cover 123. Therefore, the cover 126B results in being inclined. Thus, when a sample, etc. , used in the work space A are placed on the cover 126B, the sample, etc., roll (slide) down and move from the cover 126B onto, for example, the cover 123 under their own weight. Thus, a sample, etc., can be prevented from staying on the cover 126B. Therefore, when the cover 126B is pushed downward with the finger 300, the sample, etc. , on the cover 126B can be prevented from falling down the hole portion 125. The cover 126B may be formed, for example, through punching process using a die.

The cover 126C (Fig. 14) is formed of such a curved shape that the end portion on -Y side in the cover 126C is provided below (-Z) the cover 123. The cover 126C may be formed, for example, through punching process using a die. With this configuration, for example, an article used for work, the glove 400, etc. , can be prevented from being caught, for example, by the cover 126C protruding above the work platform 111.

### <Stopper>

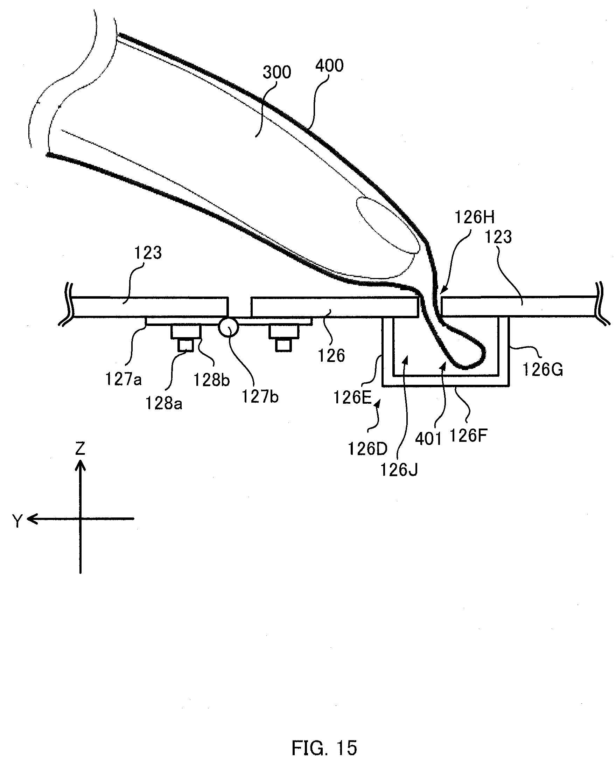

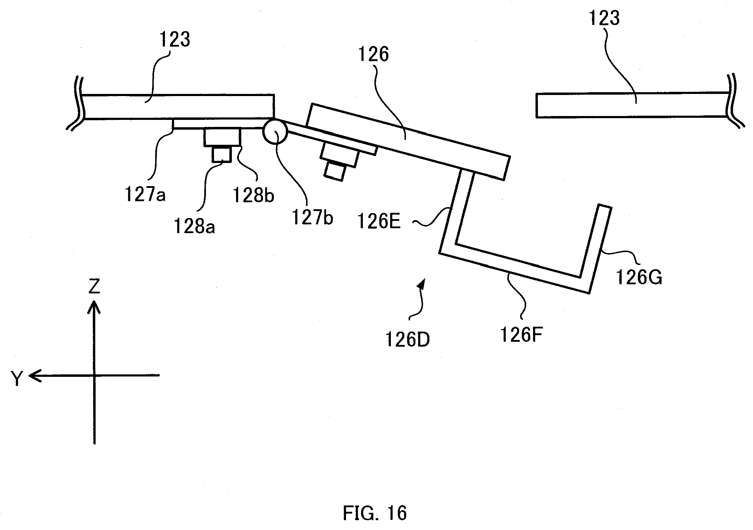

Further, in the first embodiment, it has been described that rotational movement of the mounting portions 127a is restrained with the restricting portion S (Fig. 5) abutting against the cover 123, but it is not limited thereto. For example, a stopper 126D (Figs. 15 and 16) configured to restrain rotational movement of the mounting portions 127a may be provided to the cover 126. Figs. 15 and 16 are schematic diagrams illustrating configurations of covers and a hinge according to another embodiment. Note that similar reference numerals are given to the constituents in Figs 15 and 16 which are similar to the constituents in Fig. 6, and the descriptions thereof are omitted. Fig. 15 illustrates a state where the rotational movement of the mounting portions 127a is restrained by the stopper 126D. Fig. 16 illustrates a state where the cover 126 is moved downward on the basis of a force exerted onto the cover 126 from above downward. Note that, in Fig. 16, the finger 300 and the glove 400 illustrated in Fig. 15 are omitted for the purpose of illustration.

The stopper 126D (Fig. 15) is fixed to the back surface (-Z) of the cover 126. The stopper 126D is formed, for example, by bending a sheet of metal flat plate. The stopper 126D includes a first bent piece 126E, a second bent piece 126F, and a third bent piece 126G. The upper (+Z) end portion in the first bent piece 126E is fixed to the cover 126. The second bent piece 126F and the third bent piece 126G are formed by bending from the first bent piece 126E. The first bent piece 126E, the second bent piece 126F, and the third bent piece 126G are respectively set to the lengths of such a degree as to form a space 126J in which a tip portion 401 is housed. The space 126J is a space formed when a force exerted onto the cover 126 from above downward is removed and the upper end portion of the third bent piece 126G comes into contact with the cover 123. The space 126J is a space surrounded by the cover 126, the first bent piece 126E, the second bent piece 126F, the third bent piece 126G, and the cover 123. Further, the tip portion 401 is a part of the glove 400 to be provided below the cover 126 and the cover 123 by being caught between the cover 126 and the cover 123 when the finger 300, which is inserted into the hole portion 125 (Fig. 7) while pushing down the cover 126, is pulled out from the hole portion 125. Note that the respective lengths of the first bent piece 126E, the second bent piece 126F, and the third bent piece 126G, for example, may be set experimentally on the basis of whether the tip portion 401 is caught between the third bent piece 126G and the cover 123 when performing operations of opening/closing the cover 126.

For example, when a force exerted onto the cover 126 from above downward is removed, the upper end portion of the third bent piece 126G comes into contact with the back surface of the cover 123, and thereby the mounting portion 127a is restrained from moving rotationally. Note that, it becomes possible to adjust relative positions of the cover 126 and the cover 123 when a force exerted onto the cover 126 from above downward has been removed, by adjusting the length in the Z-axis direction of the third bent piece 126G. For example, the length in the Z-axis direction of the third bent piece 126G may be adjusted such that the cover 126 and the cover 123 are provided on substantially the same plane, the length in the Z-axis direction of the third bent piece 126G may be adjusted such that the cover 126 is provided above the cover 123, and the length in the Z-axis direction of the third bent piece 126G may be adjusted such that the cover 126 is provided below the cover 123. Thus, the cover 126 and the cover 123 are to be provided on substantially the same plane, as illustrated in Fig. 15, when a force exerted onto the cover 126 from above downward has been removed. That is, the cover 126 and the cover 123 can be brought into a flat state. Therefore, when a sample, etc., to be used in the work space A are placed, the sample, etc., can be prevented from rolling to move under their own weights. Further, when a force exerted onto the cover 126 from above downward has been removed, the tip portion 401 is housed in the space 126J without being caught between the third bent piece 126G and the cover 123. Thus, when performing operations of opening/closing the cover 126, the tip portion 401 can be reliably pulled out from a gap 126H. As described above, it is possible to provide the user-friendly isolator system 100 capable of bringing the cover 126 and the cover 123 into a flat state, and preventing the glove 400 from being caught and becoming unable to be pulled out when operating the cover 126. Note that the stopper 126D may be formed by, for example, welding other members corresponding to the first bent piece 126E, the second bent piece 126F, and the third bent piece 126G.

As described above, the embodiments have been described as exemplification of the technologies in the present disclosure. Accordingly, the accompanying drawings and the detailed description have been provided.

Hence, the constituent elements described in the accompanying drawings and the detailed description may include not only constituent elements required for solving the problems, but also constituent elements not required for solving the problems, in order to exemplify the above technologies. Thus, even when those constituent elements not required are described in the accompanying drawings and the detailed description, it should not be immediately recognized that those constituent elements not required should be requirements.

Further, since the embodiments described above are to exemplify the technologies in the present disclosure, various types of modification, replacement, addition, omission and/or the like can be made in the scope of claims or in the scope equivalent thereto.

### [Industrial Applicability]

The present disclosure can be applied to laboratory environment equipment in which work is conducted with worker's hands being inserted into a box-shaped laboratory environment.

### [Reference Signs List]

- 100: isolator system
- 110: isolator
- 111: work platform
- 112: front surface plate
- 113: front surface opening portion
- 114: glove
- 115: connection opening portion
- 116: connection opening portion
- 117: partition plate
- 120: centrifuge unit
- 123: cover
- 124: hinge
- 125: hole portion
- 126: cover
- 127: hinge
- 127a: mounting portion
- 127b: axis
- 128a: bolt
- 128b: nut
- 129: spring
- 130: observation unit
- 134: cover
- 135: hinge
- 136: hole portion
- 137: cover
- 138: hinge
- 140: sterilize unit
- 150: air conditioning unit
- 150a: intake unit
- 150b: discharge unit
- 160: control unit
- 170: pass box
- 180: air conditioning unit
- 200: incubator
- 300: finger
- 400: glove

## Claims

1. An isolator attached with gloves, comprising:
a main body case including a work space isolated from an exterior thereof;
a work plate provided within the main body case, the work plate having formed therein an opening portion connecting the work space and a device provided below the main body case;
a first cover member mounted to the work plate in a manner openable and closable with respect to the opening portion, the first cover member having formed therein a hole portion into which a worker's finger is to be inserted; and
a second cover member mounted to the first cover member in a manner openable and closable with respect to the hole portion.

2. The isolator according to claim 1, comprising
an elastic member mounted to the first and the second cover members, the elastic member being configured to push upward the second cover member with respect to the first cover member, wherein
the second cover member is configured to move downward by being pushed downward with a worker's finger, and return to substantially a same plane as the first cover member when the second cover member is released from the worker's finger.

3. The isolator according to one of claims 1 and 2, wherein
the second cover member is configured to be moved, by the elastic member, about one end side of the second cover member as an axis, with respect to the first cover member.

4. The isolator according to any one of claims 1 to 3, wherein
the second cover member is arranged in such a position that a gap between the first cover member and the second cover member is greater than a thickness of a material in fingertips of the gloves.

5. The isolator according to claim 4, wherein
the second cover member is arranged in such a position that an opening width between an end portion on an other end side of the second cover member and the hole portion of the first cover member is three times or more the thickness at a fingertips of the gloves.

6. The isolator according to any one of claims 1 to 5, wherein
the first cover member and the second cover member are provided on substantially a same plane as the work plate.

7. The isolator according to any one of claims 1 to 5, wherein
the second cover member is mounted to the first cover member via the elastic member such that a part of an upper surface of the second cover member is positioned above an upper surface of the first cover member.

8. The isolator according to claim 3, wherein
the second cover member is mounted to the first cover member via the elastic member such that an upper surface on an other end side of the second cover member is positioned above an upper surface of the first cover member.

9. The isolator according to claim 7, wherein
the second cover member is mounted to the first cover member via the elastic member such that a lower surface of the second cover member is positioned below the upper surface of the first cover member.

10. The isolator according to claim 8, wherein
the second cover member is mounted to the first cover member via the elastic member such that a lower surface on the other end side of the second cover member is positioned below the upper surface of the first cover member.

11. The isolator according to claims 1 to 10, wherein
a restraint portion is provided on a lower surface of at least one of the first cover member and the second cover member, the restraint portion being configured to restrain rotational movement of the second cover member with respect to the first cover member.

12. The isolator according to claim 11, wherein
the restraint portion also serves as a part configured to fix the elastic member to the lower surface of at least one of the first cover member and the second cover member.

13. The isolator according to any one of claims 2 to 12, wherein
the elastic member includes a first portion mounted to the first cover member and a second portion mounted to the second cover member, and
the second cover member is brought into a rest state, with a part of the second portion abutting against a back surface of the first cover member.
